# EUROPÄISCHE PATENTSCHRIFT

(11) **EP 0 301 360 B1**
(45) Veröffentlichungstag und Bekanntmachung des Hinweises auf die Patenterteilung: **18.11.1993**
(21) Anmeldenummer: 88111545.5
(22) Anmeldetag: 18.07.1988
(51) Int. Cl.: A61B 17/22, G10K 9/12

(54) **Stosswellenquelle mit zentralem Ultraschall-Ortungssystem**
Shock wave apparatus with a central ultrasonic location system
Générateur d'ondes de choc avec système central de repèrage ultrasonique

(30) Priorität: 31.07.1987 DE 3725533; 19.08.1987 DE 3727691
(43) Veröffentlichungstag der Anmeldung: 01.02.1989
(73) Patentinhaber: SIEMENS AKTIENGESELLSCHAFT, 80333 München (DE)
(72) Erfinder: Grasser, Franz, D-8551 Eggolsheim (DE); Reichenberger, Helmut, Dr., D-8501 Eckental (DE); Naser, Georg, D-8502 Zirndorf (DE); Hassler, Dietrich, D-8525 Uttenreuth (DE); Schmidt, Erhard, D-8520 Erlangen (DE)

(56) Entgegenhaltungen:
- EP-A- 0 268 082
- DE-A- 3 328 039
- FR-A- 2 591 467

## Beschreibung

Die Erfindung betrifft einen Stoßwellengenerator zum berührungslosen Zertrümmern eines im Körper eines Lebewesens befindlichen Konkrements mittels Stoßwellen mit einer Stoßwellenquelle nach dem elektromagnetischen Prinzip, die einen Spulenträger, eine Flachspule und eine gegenüber der Flachspule isolierte metallische Membran umfaßt und die über ein Koppelmedium auf das Konkrement ausrichtbar ist, und mit einem Ultraschallkopf einer Ultraschall-Sende- und -Empfangseinrichtung zum Senden eines akustischen Signals und zum Empfang von Echosignalen, die durch Wechselwirkung des akustischen Sendesignals im Körper des Lebewesens entstehen, zwecks Ortung und Beobachtung des Konkrements.

In der DE-OS 33 28 051 ist ein Stoßwellengenerator beschrieben, der ein Stoßwellenrohr, bestehend aus einem Mantel und einer Stoßwellenquelle mit einer Flachspule und einer durch eine Isolierfolie getrennte Kupfermembran, aufweist. In dem Stoßwellenrohr ist eine akustische Sammellinse angeordnet, die die von der Membran erzeugten ebenen Stoßwellen in einem Brennpunkt fokussiert und die Austrittsöffnung für die Stoßwellen aus dem Stoßwellenrohr bildet. Zur Ankopplung des Stoßwellenrohres an den Patienten ist die der Membran gegenüberliegende Öffnung des Stoßwellengenerators mit einem Sack abgeschlossen, der wie das gesamte Stoßwellenrohr mit einem Koppelmedium gefüllt ist. Zur Ankopplung wird der Stoßwellengenerator so lange auf den Patienten zugefahren, bis daß das zu zerstörende Konkrement sich im Brennpunkt der Linsenanordnung befindet. Hierbei legt sich der mit der Koppelflüssigkeit gefüllte Sack an der Oberfläche des Patienten an, so daß gewährleistet ist, daß die Stoßwellen immer innerhalb der Flüssigkeit verlaufen.

In der EP-A-0 081 639 ist eine Ortungsvorrichtung für einen Stoßwellengenerator beschrieben, die aus zwei Röntgenröhren mit Bildverstärkern oder Ultraschallwandlern bestehen kann, die seitlich an dem Stoßwellengenerator angebracht sind. Dadurch ist es möglich, gleichzeitig die Zerkleinerung der Konkremente mit zu beobachten. Durch die verschiedenen Einstrahlrichtungen des Stoßwellengenerators und der Untersuchungsvorrichtung wird aber die Ortung und Einstellung erschwert. Weiterhin wird durch das seitlich angebrachte Untersuchungssystem das Gesamtsystem größer, so daß dessen Handhabung erschwert wird.

Ein Stoßwellengenerator der eingangs genannten Art ist in der DE-OS 33 28 039 in den Figuren 1 und 3 angegeben. Der Ultraschallkopf einer Ultraschall-Sende- und -Empfangseinrichtung zum Orten und Beobachten des Konkrements ist dort neben bzw. zwischen mehreren Stoßwellenquellen angeordnet. Als nachteilig ist zu werten, daß die seitliche Anordnung des Ultraschallkopfes zusätzliches Bauvolumen beansprucht. Auch kann durch diese Anordnung die Ortung und Beobachtung des Konkrements u.U. erschwert werden.

Die Verbindung einer Stoßwellenquelle mit einer Ultraschall-Ortungsvorrichtung ist auch in der EP-A-0 148 653 sowie der FR-A-2 591 467 angegeben. Hier arbeitet die Stoßwellenquelle jedoch nicht nach dem elektromagnetischen Prinzip. Sie ist vielmehr aus einer Vielzahl (z.B. 300 oder 400) piezoelektrischen Elementen gebildet und weist eine zentrale Öffnund zur Aufnahme der Ultraschallsonde eines Ultraschall-Ortungssystems auf, wobei im Falle der EP-A-0 148 653 eine feste Verbindung der Ultraschallsonde mit der Stoßwellenquelle vorliegt, während diese im Falle der FR-A-2 591 467 relativ zur Stoßwellenquelle verstellbar ist. Da im Falle der bekannten piezoelektrischen Stoßwellenquellen jedes piezoelektrische Element unabhängig von den anderen einen akustischen Impuls erzeugen kann, hat das Weglassen von einem oder mehreren piezoelektrischen Elementen keinen Einfluß auf die Funktion der Stoßwellenquelle. Es ist im Falle von piezoelektrischen Stoßwellenquellen deshalb leicht möglich, durch Weglassen eines oder mehrerer piezoelektrischer Elemente eine Öffnung zur Aufnahme der Ultraschallsonde eines Ultraschall-Ortungssystems zu schaffen.

In der älteren Anmeldung EP-A-0 268 082, die gemäß Art. 54(3) EPÜ zu berücksichtigen ist, insbesondere Fig. 3, ist vorgeschlagen, in gewissen Bauteilen einer Stoßwellenquelle zentral eine Öffnung vorzusehen, in die der Sende- und Empfangskopf eines Ultraschall-Ortungsgeräts eingebracht wird. Anders als bei der vorliegenden Erfindung müssen die Ultraschallsignale bei Emission und Empfang durch eine dämpfende Isolierfolie hindurchtreten.

Aufgabe der Erfindung ist es, einen Stoßwellengenerator zum berührungslosen Zertrümmern eines im Körper eines Lebewesens befindlichen Konkrements der eingangs genannten Art derart auszubilden, daß er klein und kompakt aufgebaut ist, eine exakte Beobachtung und/oder Lokalisation des Konkrements ermöglicht und eine einfache Handhabung gewährleistet.

Die Erfindung beruht auf der Erkenntnis, daß auch bei einer elektromagnetischen Stoßwellenquelle eine exakte Beobachtung erreicht werden kann, wenn die Symmetrieachse der Abtastebene mit der Zentralachse der Stoßwellenquelle zusammenfällt, ohne daß darunter die Stabilität des Aufbaus leidet.

Die genannte Aufgabe wird erfindungsgemäß dadurch gelöst, daß im Spulenträger, in der Flachspule und in der metallischen Membran jeweils eine zentrale Öffnung vorgesehen ist, in die der Ultraschallkopf der Ultraschall-Sende- und -Empfangseinrichtung derart einführbar ist, daß er mit dem Koppelmedium in Berührung steht. Durch die derartige Anordnung eines Ultraschallkopfes wird immer gewährleistet, daß die Ultraschallwellen und die Stoßwellen immer die gleiche Einfallrichtung aufweisen, so daß eine Zentrierung der beiden Systeme immer vorhanden ist. Weiterhin kann eine Kontrolle der Ankopplung des Sackes an dem Patienten mit Hilfe der Ultraschallwellen erfolgen. Ebenfalls wird eine kontinuierliche Mitbeobachtung der Steindesintegration ermöglicht. Weiterhin wird eine einfache Handhabung gewährleistet, da in diesem Fall nur ein System einzustellen ist. Wird weiterhin darauf geachtet, daß die Austrittsöffnung für die Stoßwellen beim Stoßwellengenerator mit integriertem Ultraschallkopf gleich groß ist wie die des Stoßwellengenerators ohne Ultraschallkopf, so wird erreicht, daß insgesamt betrachtet eine größere Einschallfläche der Stoßwellen in den Patienten erreicht wird, so daß ein mögliches Schmerzempfinden reduziert wird. Ebenfalls bewirkt die zentrische Anordnung des Ultraschallkopfes in dem Stoßwellengenerator eine Linearisierung der Stoßwellen, da das auftretende Maximum in der Mitte des Stoßwellengenerators reduziert wird.

Eine vorteilhafte Ausgestaltung der Einrichtung zeichnet sich dadurch aus, daß der Spulenträger, die Flachspule und die metallische Membran eben ausgebildet sind, und daß eine akustische Fokussierungsvorrichtung zur Fokussierung der Stoßwellen auf das Konkrement vorgesehen ist. Bei einer Weiterbildung ist in der Fokussierungsvorrichtung ebenfalls eine zentrale Öffnung vorgesehen, in die der Ultraschallkopf der Ultraschall-Sende- und -Empfangseinrichtung einführbar ist.

Eine besondere vorteilhafte Ausgestaltung der Erfindung zeichnet sich dadurch aus, daß der Ultraschallkopf in einer Halterung angeordnet ist, die in Richtung der Zentralachse der Stoßwellenquelle ausgerichtet ist, und daß die Halterung um die Zentralachse drehbar ist.

Weitere Ausbildungen und Vorteile der Erfindung ergeben sich aus der nachfolgenden Beschreibung von Ausführungsbeispielen anhand der Zeichnung sowie aus den Unteransprüchen. Es zeigen:
Fig. 1 einen erfindungsgemäßen Stoßwellengenerator mit elektromagnetischer Stoßwellenquelle, und
Fig. 2 eine weitere Ausführungsform eines erfindungsgemäßen Stoßwellengenerators.

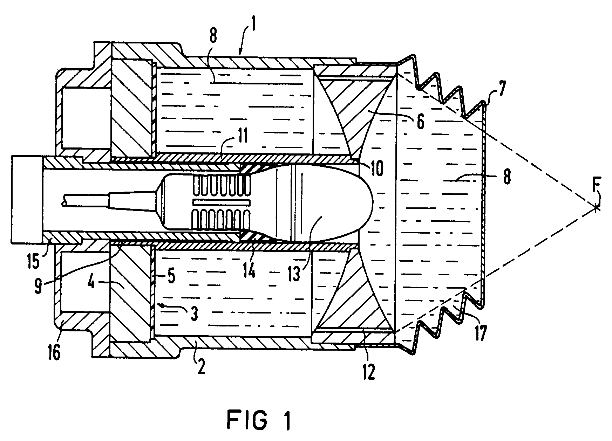

In der Fig. 1 ist ein Stoßwellengenerator 1 mit einem mit einer Flüssigkeit, z.B. Wasser, gefülltem Stoßwellenrohr 2 dargestellt, dessen eine Öffnung durch eine Stoßwellenquelle 3, bestehend aus einem Spulenträger 4 mit einer Flachspule und einer gegenüber der Flachspule durch eine Isolierfolie getrennte Membran 5, die durch einen Rückhaltering eng aneinander gepreßt zusammengehalten werden, abgeschlossen wird. Die andere Öffnung des Stoßwellenrohres 2 wird durch eine Linsenanordnung 6 mit der Funktion einer akustischen Sammellinse abgedeckt. Das Stoßwellenrohr 2 und die durch die Linsenanordnung 6 gebildete Austrittsöffnung für die Stoßwellen werden von einem Ankoppelsack 7 abgedeckt, so daß sich ein Ankopplungsraum 17 bildet, der mit einem Koppelmedium 8 gefüllt ist. Im angekoppelten Zustand liegt der Ankoppelsack 7 an der Haut eines Patienten an. Dann befindet sich beispielsweise ein in einer Niere vorhandenes Konkrement in dem Brennpunkt F der Linsenanordnung 6, so daß es durch vom Stoßwellengenerator 1 erzeugte Stoßwellen zerstört werden kann.

Die Stoßwellenquelle 3 und die Linsenanordnung 6 des Stoßwellengenerators 1 weisen je eine zentrische Öffnung 9 und 10 auf, wobei beide Öffnungen 9 und 10 durch ein Rohr 11 verbunden sind. Der noch verbleibende Raum zwischen Stoßwellenrohr 2, Rohr 11, Stoßwellenquelle 3 und Linsenanordnung 6 ist ebenfalls mit dem Koppelmedium gefüllt, die durch in der Linsenanordnung 6 angebrachte Kanäle 12 mit dem Koppelmedium 8 des Ankoppelsackes 7 in Verbindung steht.

In das Rohr 11 ist von der Seite der Stoßwellenquelle 3 her ein Ultraschallkopf 13 eingeführt, der teilweise in den durch die Linsenanordnung 6 und den Ankoppelsack 7 begrenzten Ankopplungsraum 17 ragt. Von der Seite der Stoßwellenquelle 3 her ist der Ultraschallkopf 13 mit einer Dichtung 14 versehen, die den Ultraschallkopf 13 gegenüber das Rohr 11 abdichtet, so daß kein Koppelmedium 8 entweichen kann. Die Dichtung 14 und der hintere Teil des Ultraschallkopfes 13 werden durch eine rohrförmige Halterung 15 arretiert, die mit einer die Stoßwellenquelle 3 abdeckenden Abdeckkappe 16 verbunden ist.

Die Stoßwellenquelle 3 des Stoßwellengenerators 1 erzeugt in bekannter Weise ebene Stoßwellen, die durch die akustische Linse 6 auf den Brennpunkt F fokussiert werden. Da der Ultraschallkopf 13, beispielsweise ein Sektorscanner, zentrisch in dem Stoßwellengenerator 1 angeordnet ist, ist seine Zentrumsachse somit ebenfalls auf den Brennpunkt F ausgerichtet, so daß unabhängig von der Drehrichtung des Ultraschallkopfes 13 und damit der Richtung des Ultraschallfächers der Brennpunkt F immer von den Ultraschallwellen erfaßt wird. Dadurch ist es möglich, durch Drehen des Ultraschallkopfes 13 das Konkrement vom Ultraschallfächer erfassen, so daß es auf einem nicht dargestellten Monitor sichtbar ist. Anschließend läßt sich der Stoßwellengenerator 1 in Scanrichtung derart verschieben, bis sich das Konkrement im Brennpunkt F des Stoßwellengenerators 1 befindet, der auf der Zentrumsachse des Ultraschallkopfes 13 liegt.

Bei der Einstellung des Stoßwellengenerators 1 läßt sich gleichzeitig die Ankopplung des Ankoppelsackes 7 an den Patienten kontrollieren, so daß immer eine optimale Ankopplung gewährleistet ist. Weiterhin läßt sich mit einem derartigen Stoßwellengenerator 1 mit integriertem Ultraschallkopf 13 der Erfolg der Steindesintegration kontinuierlich mitbeobachten. Auch läßt sich ein derartiger Stoßwellengenerator 1 für in der Gallenblase befindliche Gallensteine einsetzen, die für Röntgenstrahlen transparent sind.

Durch diese erfindungsgemäße Vorrichtung erhält man einen Stoßwellengenerator, mit dem sich die Zertrümmerung von Konkrementen innerhalb eines Patienten, beispielsweise Nieren- oder Gallensteine, auf einfache Weise beobachten läßt.

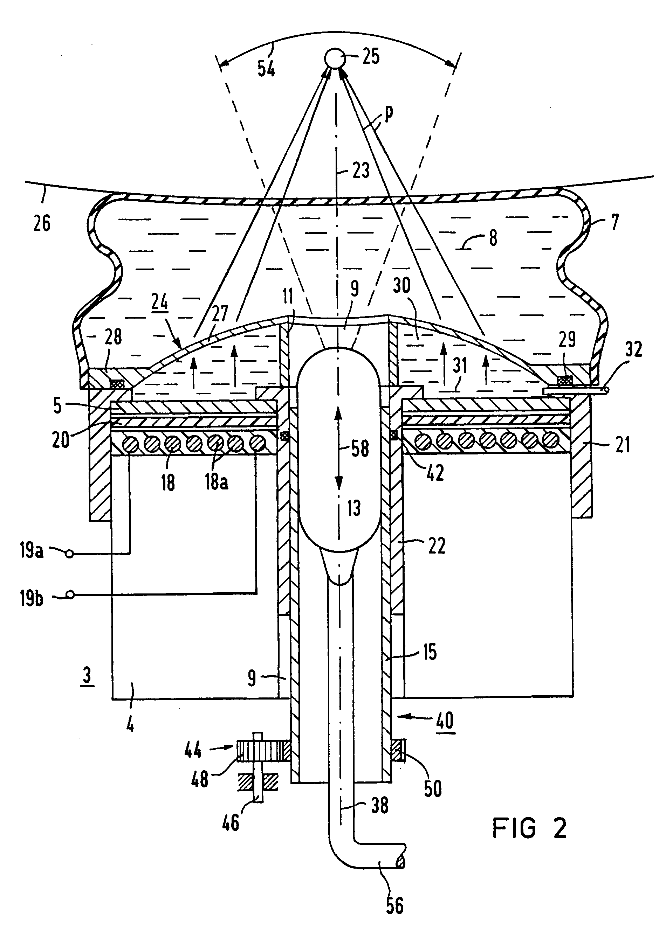

In der Figur 2 ist der Spulenträger 4 dargestellt, der die äußere Form eines Zylinders besitzt. Im Spulenträger 4 ist zentral in Richtung der Längsachse eine zylinderförmige Öffnung 9 eingebracht. Der Spulenträger 4 besteht bevorzugt aus einem keramischen Material. An einer planen Seite des Spulenträgers 4 ist eine ebene Flachspule 18 befestigt. Die spiralförmigen, in einem Kunststoff-Guß untergebrachten Windungen 18a der Flachspule 18 nehmen die gesamte ebene Seite des Spulenträgers 4 ein. Die beiden elektrischen Anschlüsse der Flachspule 18 sind mit 19a und 19b bezeichnet. Die Flachspule 18 weist ebenso wie der Spulenträger 4 eine zentrale Öffnung 9 auf.

An der Flachspule 18 ist auf der dem Spulenträger 4 gegenüberliegenden Seite eine Isolierfolie 20 befestigt. Die Isolierfolie 20 weist ebenfalls eine zentrale Öffnung 9 auf. Sie ist bevorzugt auf die Flachspule 18 geklebt. Ohne jeden Abstand zur Isolierfolie 20 ist die kreisringförmige metallische Membran 5 angeordnet, die ebenfalls eine zentrale Öffnung 9 aufweist. Der enge Kontakt zwischen der Isolierfolie 20 und der Membran 5 wird in bekannter Weise durch einen Unterdruck sichergestellt. Die zentrale Öffnung 9 der Membran 5 ist genauso groß wie die zentrale Öffnung 9 des Spulenträgers 4, der Flachspule 18 und der Isolierfolie 20.

Der Spulenträger 4, die Flachspule 18 mit der aufgeklebten Isolierfolie 20 und die metallische Membran 5 sind an ihrem äußeren Rand mit einer ersten ringförmigen Einspannung 21 fest verbunden. In ähnlicher Weise sind diese Teile 4, 5, 18 und 20 an den zentralen Öffnungen 9 mit einer zweiten ringförmigen oder rohrförmigen Einspannung 22 fest verbunden. Die Einspannungen 21 und 22 stellen sicher, daß die metallische Membran 5 randseitig festgehalten, d.h. unbeweglich zur Flachspule 18 ist.

Der Spulenträger 4, die Flachspule 18 mit der Isolierfolie 20, die metallische Membran 5 und die Einspannungen 21 und 22 sind das Kernstück der elektromagnetischen Stoßwellenquelle 3. Die Zentralachse 23 der Stoßwellenquelle 3 fällt zusammen mit den Mittelpunkten der Öffnungen 9. Fließt ein sich schnell ändernder Strom (steiler Stromimpuls von einer Kondensatorentladung) über die Anschlüsse 19a und 19b durch die Flachspule 18, dann wird dadurch in der metallischen Membran 5 ein Strom induziert. Der Strom in der Flachspule 18 und der Strom in der Membran 5 erzeugen jeweils ein derartiges Magnetfeld, daß die metallische Membran 5 von der Flachspule 6 abgestoßen wird. Die metallische Membran 5 wird zwischen den Einspannungen 21 und 22 nach außen ausgelenkt und erzeugt damit einen akustischen Impuls, der sich anschließend zu einer Stoßwelle p formt.

An die elektromagnetische Stoßwellenquelle 3 ist eine Fokussierungsvorrichtung 24 angeschlossen. Diese ist hier eine plankonvex geformte akustische Flüssigkeitslinse, die die im wesentlichen ebenen akustischen Impulse auf ein Konkrement 25 im Patienten 26 fokussiert, z.B. auf einen Nieren- oder Gallenstein. Die akustische Linse 24 besitzt ebenfalls eine zentrale Öffnung 9. Die plane rückseitige Fläche der akustischen Linse 24 wird von der metallischen Membran 5 gebildet. Die konvexe Fläche ist durch eine kalottenförmige Kappe 27 gebildet. Diese besteht aus einem formstabilen Kunststoff, bevorzugt aus Polystyrol. Es kommt auch Polymethylmetacrylat (PMMA) oder Polyäthylen in Betracht. Die Kappe 27 besitzt also ebenfalls eine zentrale Öffnung 9, die mit den anderen Öffnungen 9 fluchtet.

Die Kappe 27 geht randseitig in einen Anschlußflansch 28 über. Mit ihrem Anschlußflansch 28 ist die Kappe 27 an der ersten Einspannung 21 befestigt. Die Befestigung geschieht z.B. mit Hilfe von Schrauben (nicht gezeigt); die Kappe 27 kann jedoch auch mit dem Anschlußflansch 28 an die erste Einspannung 21 geklebt sein. Zweckmäßigerweise wird zur Abdichtung ein O-Ring 29 verwendet. Zwischen der zentralen Öffnung 9 der Kappe 27 und der zweiten Einspannung 22 befindet sich ein Rohr 11. Das Rohr 11 ist z.B. mit einem Ende mit der Kappe 27 und mit seinem anderen Ende mit der zweiten Einspannung 22 verklebt. Innerhalb der akustischen Linse 24 ist somit ein Innenraum 30 ausgebildet, der begrenzt ist von der Membran 5, der Kappe 27, dem Rohr 11 und den Einspannungen 21 und 22. Der Innenraum 30 ist über eine Leitung 32 mit einer Flüssigkeit 31 füllbar. Es kann eine weitere Öffnung (hier nicht gezeigt) vorhanden sein, die einen Kreislauf der Flüssigkeit 31 erlaubt.

Die Abdichtung des Innenraums 30 ist hier, wie erwähnt, durch den O-Ring 29 gewährleistet, der sich in einer Nut im Anschlußflansch 28 befindet.

Am Rand des Anschlußflansches 28 ist der Ankoppelsack 7 befestigt, der mit dem Koppelmedium 8, z.B. mit einer Flüssigkeit wie entgastem Wasser, gefüllt ist. Er erlaubt eine gute akustische Ankopplung der Stoßwellenquelle 3 an das zu behandelnde Lebewesen 26.

Die Flüssigkeit 31 im Innenraum 30 der akustischen Linse 24 hat die Eigenschaft, daß ihre Schallgeschwindigkeit deutlich geringer ist als die Schallgeschwindigkeit von Wasser. Die Flüssigkeit ist bevorzugt eine halogenierte Kohlenwasserstoffverbindung, wie z.B. Tetrachlorkohlenstoff, oder ein komplett fluorierter Kohlenwasserstoff. Ebenso sind Silikone verwendbar.

Innerhalb der zweiten Einspannung 16 befindet sich die rohrförmige Halterung 15. Die Halterung 15 ist um ihre Längsachse 38 drehbar gelagert. Die Längsachse 38 fällt mit der Zentralachse 23 der Stoßwellenquelle 3 zusammen. Sie ist Teil einer wasserdichten Drehdurchführung 40. Zur Drehdurchführung 40 gehören neben der Halterung 15 haupsächlich noch eine Abdichtung 42 und ein Drehantrieb 44. Die Abdichtung 42 besteht aus einem O-Ring, der sich in einer Nut außen auf der zweiten Einspannung 22 befindet. Die Abdichtung 42 verhindert, daß Wasser aus dem Ankoppelsack 7 über die Drehdurchführung 40 austreten kann. Der Drehantrieb 44 setzt sich zusammen aus einer Antriebswelle 46, an deren Ende ein Zahnrad 48 befestigt ist, und aus einem Zahnkranz 50, der auf der Halterung 15 in gleicher Höhe wie das Zahnrad 48 befestigt ist. Der Zahnkranz 50 kann sich über eine volle Umfangslinie oder aber nur über einen Teil derselben erstrecken.

Die Drehdurchführung 40 ist gegen axiales Verschieben gesichert, was hier jedoch nicht weiter gezeigt ist.

In der Halterung 15 ist der Ultraschallkopf 13 einer konventionellen Ultraschall-Sende- und -Empfangseinrichtung flüssigkeitsdicht angeordnet. Der Ultraschallkopf 13 ist bevorzugt ein Sektorscan-Applikator. Der Winkel des Abtastsektors ist durch einen gekrümmten Doppelpfeil 54 angedeutet. Die Längsachse des Ultraschallkopfs 13 und die Zentralachse 19 der Stoßwellenquelle 18 fallen zusammen.

Der Ultraschallkopf 13 ist über eine elektrische Leitung 56 mit der Ultraschall-Sende- und -Empfangseinrichtung verbunden. Endseitig steht der Ultraschallkopf 13 mit dem Koppelmedium 8 in Berührung. Der Ultraschallkopf 13 ist innerhalb der Halterung 15 in Richtung der Zentralachse der Stoßwellenquelle verschiebbar; dies ist durch einen geraden Doppelpfeil 58 angedeutet. Dazu kann der Ultraschallkopf 13 z.B. in einer (hier nicht gezeigten) weiteren Halterung befestigt sein, die ihrerseits in der Halterung 15 verschiebbar angeordnet ist.

Die in den Figuren gezeigte Einrichtung ist in bekannter Weise an einem Haltearm oder Stativ befestigt, was hier jedoch nicht weiter dargestellt ist. Der Haltearm ermöglicht eine genaue Positionierung und Fixierung der Stoßwellenquelle 3 am Körper des Lebewesens 26.

Die durch die Öffnungen 9 bewirkte Einbuße an akustischer Leistung der Stoßwellenquelle 3 kann toleriert werden, da einerseits die zentrale Region der elektromagnetischen Quelle 3 relativ wenig zur gesamten abgegebenen Leistung beiträgt und andererseits der zentrale Teil der Flüssigkeitslinse 24 am meisten dämpfend wirkt. Durch Verwendung eines Sektorscan-Applikators 13 ist eine genaue, hochaufgelöste Betrachtung des gesamten vor der Stoßwellenquelle 3 liegenden Gebietes sowie eine genaue Ausrichtung der Quelle 3 auf das Konkrement 25 möglich.

Die weiteren Vorteile dieser Einrichtung sind eine kompakte Bauform und eine exakte Ultraschall-Betrachtung des mit Stoßwellen p behandelten Gebietes 25 direkt aus der Richtung dieser Stoßwellen p.

## Patentansprüche

1. Stoßwellengenerator (1) zum berührungslosen Zertrümmern eines im Körper eines Lebewesens befindlichen Konkrements (25) mittels Stoßwellen mit einer Stoßwellenquelle (3) nach dem elektromagnetischen Prinzip, die einen Spulenträger (4), eine Flachspule (18) und eine gegenüber der Flachspule (18) isolierte metallische Membran (5) umfaßt und die über ein Koppelmedium (8) auf das Konkrement (25) ausrichtbar ist, und mit einem Ultraschallkopf (13) einer Ultraschall-Sende- und -Empfangseinrichtung zum Senden eines akustischen Sendesignals und zum Empfang von Echosignalen, die durch Wechselwirkung des akustischen Sendesignals im Körper des Lebewesens entstehen, zwecks Ortung und Beobachtung des Konkrements (25), wobei im Spulenträger (4), in der Flachspule (18) und in der metallischen Membran (5) jeweils eine zentrale Öffnung (9) vorgesehen ist, in die der Ultraschallkopf (13) der Ultraschall-Sende- und -Empfangseinrichtung derart einführbar ist, daß er mit dem Koppelmedium (8) in Berührung steht.

2. Stoßwellengenerator (1) nach Anspruch 1, **dadurch gekennzeichnet,** daß der Spulenträger (4), die Flachspule (18) und die metallische Membran (5) eben ausgebildet sind und daß eine akustische Fokussierungsvorrichtung (6, 24) zur Fokussierung der Stoßwellen auf das Konkrement (25) vorgesehen ist.

3. Stoßwellengenerator (1) nach Anspruch 2, **dadurch gekennzeichnet,** daß in der Fokussierungsvorrichtung (6, 24) ebenfalls eine zentrale Öffnung (9, 10) vorgesehen ist, in die der Ultraschallkopf (13) der Ultraschall-Sende- und -Empfangseinrichtung einführbar ist.

4. Stoßwellengenerator (1) nach einem der Ansprüche 1 bis 3, **dadurch gekennzeichnet,** daß der Ultraschallkopf (13) in einer Halterung (15) angeordnet ist, die in Richtung der Zentralachse (23) der Stoßwellenquelle (3) ausgerichtet ist.

5. Stoßwellengenerator (1) nach Anspruch 4, **dadurch gekennzeichnet,** daß die Halterung (15) flüssigkeitsdicht ausgebildet ist.

6. Stoßwellengenerator (1) nach Anspruch 4 oder 5,**dadurch gekennzeichnet,** daß die Halterung (15) um die Zentralachse (23) drehbar ist.

7. Stoßwellengenerator (1) nach Anspruch 4, 5 oder 6, **dadurch gekennzeichnet,** daß die Halterung (15) ein zylindrisches Rohrstück (15) umfaßt, das mittels eines Drehantriebs (44) um seine Längsachse (38) drehbar ist.

8. Stoßwellengenerator (1) nach einem der Ansprüche 4 bis 7, **dadurch gekennzeichnet,** daß die Halterung (15) innerhalb einer zweiten Einspannung (22) für den Spulenträger (4), für die Flachspule (18) und für die Membran (5) angeordnet ist.

9. Stoßwellengenerator (1) nach einem der Ansprüche 1 bis 8, mit einem Ankoppelsack (7), der mit dem Koppelmedium gefüllt ist, **dadurch gekennzeichnet,** daß der Ultraschallkopf (13) endseitig mit dem Koppelmedium (8) in Berührung steht.

10. Stoßwellengenerator (1) nach einem der Ansprüche 1 bis 9, **dadurch gekennzeichnet,** daß der Ultraschallkopf (13) in Richtung der Zentralachse (23) der Stoßwellenquelle (3) verschiebbar ist.

## Claims

1. Shock wave generator (1) for the contactless disintegration by means of shock waves of a calculus (25) located in the body of a living being, having a shock wave source (3) which operates on the electromagnetic principle and comprises a coil carrier (4), a flat coil (18) and a metal membrane (5) insulated from the flat coil (18), and which can be directed at the calculus (25) by way of a coupling medium (8), and having an ultrasound head (13) of an ultrasound transmission and reception system for transmitting an acoustic transmission signal and for receiving echo signals which occur in the body of the living being by interaction of the acoustic transmission signal, for the purpose of locating and observing the calculus (25), whereby in the coil carrier (4), the flat coil (18) and the metal membrane (5) there is provided a respective central opening (9) into which the ultrasound head (13) of the ultrasound transmission and reception system can be inserted in such a way that it is in contact with the coupling medium (8).

2. Shock wave generator (1) according to claim 1, characterised in that the coil carrier (4), the flat coil (18) and the metal membrane (5) are formed flat and in that an acoustic focusing device (6, 24) is provided for focusing the shock waves on the calculus (25).

3. Shock wave generator (1) according to claim 1, characterised in that, in the focusing device (6, 24) there is also provided a central opening (9, 10) into which the ultrasound head (13) of the ultrasound transmission and reception device can be inserted.

4. Shock wave generator (1) according to one of claims 1 to 3, characterised in that the ultrasound head (13) is arranged in a mounting (15) which is aligned in the direction of the cental axis (23) of the shock wave source (3).

5. Shock wave generator according to claim 4, characterised in that the mounting (15) is formed liquid-tight.

6. Shock wave generator (1) according to claim 4 or 5, characterised in that the mounting (15) can be rotated around the central axis (23).

7. Shock wave generator (1) according to claim 4, 5 or 6, characterised in that the mounting (15) comprises a cylindrical tube section (15) which can be rotated around its longitudinal axis (38) by means of a rotary drive (44).

8. Shock wave generator (1) according to one of claims 4 to 7, characterised in that the mounting (15) is arranged within a second clamp (22) for the coil carrier (4), the flat coil (18) and the membrane (5).

9. Shock wave generator (1) according to one of claims 1 to 8, having a coupling bag (7) which is filled with the coupling medium, characterised in that the end of the ultrasound head (13) is in contact with the coupling medium (8).

10. Shock wave generator (1) according to one of claims 1 to 9, characterised in that the ultrasound head (13) can be displaced in the direction of the central axis (23) of the shock wave source (3).

## Revendications

1. Générateur d'ondes de choc (1) pour fragmenter sans contact une concrétion (25) se trouvant dans le corps d'un être vivant, au moyen d'ondes de choc, comportant une source d'ondes de choc (3) basée sur le principe électromagnétique et qui comprend un porte-bobine (4), une bobine plate (18) et une membrane métallique (5) isolée par rapport à la bobine plate (18) et qui peut être dirigée, à travers un milieu de couplage (8), sur la concrétion (25), et comportant une tête à ultrasons (13) d'un dispositif d'émission et de réception d'ultrasons pour l'envoi d'un signal d'emission acoustique et la réception de signaux d'échos, qui apparaissent sous l'effet de l'interaction du signal d'émission acoustique dans le corps de l'être vivant, en vue de repérer et d'observer la concrétion (25), et dans lequel il est prévu, dans le porte-bobine (4), dans la bobine plate (18) et dans la-membrane métallique (5), respectivement une ouverture centrale (9), dans laquelle la tête à ultrasons (13) du dispositif d'émission et de réception d'ultrasons peut être insérée de telle sorte qu'elle vient en contact avec le milieu de couplage (8).

2. Générateur d'ondes de choc (1) suivant la revendication 1, caractérisé par le fait que le porte-bobine (4), la bobine plate (18) et la membrane métallique (5) sont plats et qu'il est prévu un dispositif de focalisation acoustique (6,24) pour focaliser les ondes de choc sur la concrétion (25).

3. Générateur d'ondes de choc (1) suivant la revendication 2, caractérisé par le fait que dans le dispositif de focalisation (6, 24), il est également prévu une ouverture centrale (9,10), dans laquelle peut être insérée la tête à ultrasons (13) du dispositif d'émission et de réception d'ultrasons.

4. Générateur d'ondes de choc (1) suivant l'une des revendications 1 à 3, caractérisé par le fait que la tête à ultrasons (13) est disposée dans un support (15), qui est orienté dans la direction de l'axe central (23) de la source d'ondes de choc (3).

5. Générateur d'ondes de choc (1) suivant la revendication 4, caractérisé par le fait que le support (15) est agencé de manière à être étanche aux liquides.

6. Générateur d'ondes de choc (1) suivant la revendication 4 ou 5, caractérisé par le fait que le support (15) peut tourner autour de l'axe central (23).

7. Générateur d'ondes de choc (1) suivant la revendication 4, 5 ou 6, caractérisé par le fait que le support (15) comprend un corps tubulaire cylindrique (15), qui peut être entraîné en rotation autour de son axe longitudinal (38) par l'intermédiaire d'un dispositif d'entraînement en rotation (44).

8. Générateur d'ondes de choc (1) suivant l'une des revendications 4 à 7, caractérisé par le fait que le support (15) est disposé à l'intérieur d'un second dispositif de serrage (22) pour le porte-bobine (4), pour la bobine plate (18) et pour la membrane (5).

9. Générateur d'ondes de choc (1) suivant l'une des revendications 1 à 8, comportant un sac de couplage (7), qui est rempli par le milieu de couplage, caractérisé par le fait que la tête à ultrasons (13) est en contact par une extrémité avec le milieu de couplage (8).

10. Générateur d'ondes de choc (1) suivant l'une des revendications 1 à 9, caractérisé par le fait que la tête à ultrasons (13) est mobile dans la direction de l'axe central (23) de la source d'ondes de choc (3).
